# EUROPÄISCHE PATENTSCHRIFT

(11) **EP 2 683 418 B1**
(45) Veröffentlichungstag und Bekanntmachung des Hinweises auf die Patenterteilung: **20.01.2016**
(21) Anmeldenummer: 12708136.2
(22) Anmeldetag: 09.03.2012
(51) Int. Cl.: A61L 15/40, A61L 15/44, A61L 26/00, A61F 13/08

(54) **PRODUKTE ZUR BAKTERIENTOXINBINDUNG UND ELIMINATION BEI DER BEHANDLUNG VON ÖRTLICHEN INFEKTIONEN UND IHRE HERSTELLUNG**
PRODUCTS FOR BACTERIAL TOXIN BINDING AND ELIMINATION IN THE TREATMENT OF LOCAL INFECTIONS, AND PRODUCTION THEREOF
PRODUITS PERMETTANT LA LIAISON DE TOXINES BACTÉRIENNES ET LEUR ÉLIMINATION LORS DU TRAITEMENT D'INFECTIONS LOCALES, ET LEUR PRÉPARATION

(30) Priorität: 11.03.2011 DE 102011005449
(43) Veröffentlichungstag der Anmeldung: 15.01.2014
(73) Patentinhaber: SeBo GmbH, 69117 Heidelberg (DE)
(72) Erfinder: SEIDEL, Dietrich, 82340 Feldafing (DE)
(74) Vertreter: Weickmann & Weickmann
(86) Internationale Anmeldenummer: PCT/EP2012/054120
(87) Internationale Veröffentlichungsnummer: WO 2012/123363

(56) Entgegenhaltungen:
- DE-A1- 4 209 988
- GB-A- 1 521 171
- GB-A- 2 442 519
- US-A- 5 098 417

## Beschreibung

Die vorliegende Erfindung betrifft ein Material zur Behandlung von lokalen bakteriellen Infektionen sowie die Verwendung dieses Materials im Wundmanagement.

Infektionen allgemein, aber ganz besonders örtliche, chronisch-infizierte Wunden stellen in der Geschichte der Heilkunst seit ihrem Beginn (Hippokrates, 460-370 v. Chr.) und bis heute ein ernsthaftes medizinisches Problem dar, das auch eine gesellschaftliche Dimension erhält, wenn man bedenkt, dass alleine in Deutschland etwa 2 - 3 Millionen Menschen unter schlecht heilenden bzw. nicht heilenden Wunden leiden. Zu dem persönlichen Leid betroffener Patienten, das sich über viele Jahre erstrecken kann, sind es bemerkenswerte ca. 2 % des Gesamtgesundheitsbudgets, die in Deutschland jährlich für örtlich begrenzte Infektionen und schlecht heilende Wunden aufgebracht werden. ⁽¹⁾ In den USA beläuft sich der Aufwand hierfür auf ca. 40 Milliarden US Dollar pro Jahr. Bei einer steigenden Lebenserwartung von ca. 2 bis 3 Monaten pro Jahr wird sich dieses Problem - ohne messbaren Fortschritt in der Behandlung - weltweit noch weiter verstärken.

Die Wundheilung ganz allgemein, aber besonders die im kutanen Bereich, zählt zu den komplexesten Vorgängen des menschlichen Körpers überhaupt.^{(1,2,3)}

Normalerweise erfolgt die Wundheilung in bestimmten Phasen, die aber vielfältig gestört und in ihrem Ablauf verzögert werden können, bis hin zur Entwicklung chronisch nicht heilender Wunden. In der ersten, der exsudativinflammatorischen Phase leitet sich der Verlauf zur Heilung oder zu einer gestörten Heilung ein. Diesem ersten Heilungsabschnitt folgen dann, wenn er erfolgreich verläuft, die proliferative Phase und später die Phase des Tissue Remodeling. ^{(1,2)}

Die Behandlung von tiefliegenden, aber auch von oberflächlichen, besonders von kutanen Wunden hat sich in den letzten zwei Dekaden deutlich geändert. So ist ein Austrocknen von Wunden unter der Vorstellung der Vermeidung von Infektionen überholt, da dieses zu Störungen der Gewebereparatur und somit zu einer verlangsamten Wundheilung führt. Ziel der heutigen modernen Wundtherapie ist es vielmehr, ein physiologisches Wachstums- und Heilungsmilieu im Bereich der Wunde zu garantieren, um hierdurch pathologische Wundheilungsmechanismen und -prozesse zu verhindern. ^{(2,3,4)}

Ein erster Schritt hierbei war die Einführung von hydroaktiven Wundauflagen zur Regulation des Feuchtigkeitshaushalts im Wundgebiet. Trotz der großen Vielfalt der zu diesem Zweck angebotenen Produkte von Wundauflagen und unterschiedlichsten Drainagen (Endosponagen) bis hin zur Vakuumbehandlung ist die Überlegenheit einzelner über andere bislang aber nicht evidenzbasiert nachgewiesen. ^{(5,6)} So gibt es bislang auch noch keine verbindlichen Leitlinien zur Standardisierung eines modernen Wundmanagement. Dieses erklärt sich zum Teil dadurch, dass jede Wundbehandlung ihrer Komplexität wegen grundsätzlich individualisiert zu sein hat und auf den jeweiligen Patienten anzupassen ist.

Eine Wunde ist definiert als der Verlust der Integrität eines Organs durch exogene oder endogene Einflüsse. Abhängig von der Art der Wunde, von lokalen Wundfaktoren, von systemischen Mediatoren und zugrundeliegenden Ursachen (Verletzungen, Verbrennungen etc.), kann die Wundbehandlung einen interdisziplinären therapeutischen Ansatz zwingend erfordern.

Ist der physiologische Ablauf der Wundheilung besonders in der Phase der Exsudation und beginnenden Granulation gestört, entwickelt sich zumeist auf dem Boden einer prolongierten Entzündungsreaktion eine chronische Wunde. Das physiologische Exsudat einer Wunde ist in der Regel durch die Aktivierung von Makrophagen und Fibroblasten gekennzeichnet, durch signifikant erhöhte Konzentrationen von Zytokinen bzw. Chemokinen (IL-1, IL-6, IL-8, TNF-α u.a.) durch Proteasen und Elastasen, sowie durch Wachstumsfaktoren (FDGF, PDGF), die zusammen für die ungestörte Proliferation von Fibroblasten zur Wundabdeckung notwendig sind. ⁽³⁾

Eine mikrobielle Besiedelung der Wunde führt nachhaltig zur Störung des physiologischen Milieus und beeinflusst darüber hinaus den immunologischen Status in loco. Dieses hat zu einer immer intensiveren Anwendung von Antiseptika (Silber, Jod u. a.) sowie zu einer fast ungebremsten Verabreichung von Antibiotika bei der Wundbehandlung geführt. Es war aber zu erwarten und ist unverkennbar, dass die unkontrollierte Anwendung solcher Substanzen durch ihre Zell- und Organtoxizität auch zu erheblichen medizinischen Problemen ^{(7,8)} und vor allem zur Entwicklung multiresistenter Bakterienstämme (MRSA, ORSA oder VRE) führt. Darüber hinaus provozieren die erforderlich hohen Gewebespiegel solcher Substanzen häufig unerwünschte Wirkungen bis hin zu schweren Allergien zur Zelltoxizität und schweren Organschäden. ^{(9,10)} So besteht heute ein allgemeiner Konsens darüber, dass die Wirksamkeit dieser Substanzen nur dann aufrechterhalten bleiben kann, wenn deren Umgang sensibel kontrolliert und deren Einsatz nicht langfristig oder prophylaktisch betrieben wird.

Von überragender pathophysiologischer Bedeutung bei der Entwicklung chronisch infizierter Wunden, seien sie oberflächlich, tief oder im abgekapselten Fokus gelegen, sind meist nicht die Bakterien selbst, sondern vielmehr deren Toxine, die durch Abtötung der Bakterien entstehen und negative bis schwere lokale sowie systematische Reaktionen auslösen können. In Abhängigkeit vom Immunstatus des Patienten und der Konzentration der Bakterientoxine üben diese nicht nur einen negativen Einfluss auf die Wundheilung selbst aus, sondern unterhalten die lokalen Entzündungsprozesse und können sich in dem Gesamtkörper ausbreiten, was oft sehr schnell zu einem Organversagen, einem Multiorganversagen bis hin zum septischen Schock führen kann. An dieser Komplikation sterben auch heute noch nahezu 50 % aller betroffener Patienten. ⁽¹¹⁾ Bereits 100 ng eines bakteriellen Toxins können beim Menschen erhebliche Wirkungen zeigen, wenige Mikrogramm können dessen Tod herbeiführen.

Durch die typische Besiedelung Gram-positiver als auch Gram-negativer Bakterien auf der Körperoberfläche kommt es bei Hautverletzungen und -verbrennungen zu Komplikationen, die insbesondere durch Staphylococcus aureus oder Pseudomonas aeruginosa hervorgerufen werden.

In loco führt die Anwesenheit von bakteriellen Toxinen (wie Lipopolysaccharide [LPS] gram-negativer Bakterien und/oder Lipoteichonsäuren [LTA] gram-positiver Bakterien meist in Kombination) neben der Aktivierung der Gerinnungskaskade, der Beeinträchtigung der Fibrinolyse auch zur Freisetzung von lysosomalen Enzymen und von Peroxid-Radikalen. Dies resultiert in einer massiven Verschiebung des Mediatoren-Gleichgewichts im Wundexsudat. In den komplexen Entzündungsvorgängen und Reparationsprozessen kommt den Zytokinen, den Immunmodelatoren und den Wachstumsfaktoren gemeinsam eine bedeutende regulative Funktion zu. Erhöhte Konzentrationen an Bakterientoxinen führen zur Aktivierung von Monozyten, Endothelzellen und neutrophilen Granulozyten. In der Folge kommt es zu einer vermehrten Expression von proinflammatorischen und immunmodulatorischen Zytokinen (IL-1β, TNF-α), weiterer Entzündungsmediatoren (Histamin, Kininen, Stickstoffoxiden) sowie zu einem Apoptose-Ungleichgewicht zugunsten der Apoptose-fördernden Mitglieder der Bcl-2 Gruppe. ^{(2,3,12)} Dieses erklärt die negative Wirkung von Bakterientoxinen auf das Wundwachstum von Fibroblasten wie auch auf die Reduktion der Blutzufuhr und damit auf die Negativbilanz der Sauerstoffversorgung der infizierten Wunde. ^{(2,3)}

Die Besiedelung einer Wunde besonders mit Problemerregern verzögert also nicht nur die Wundheilung durch die direkten Folgen wie Eiterbildung oder Gewebezersetzung, sondern und besonders auch durch die Freisetzung von LPS und LTA - als Folge der antibakteriellen und bakteriziden Therapie - den gesamten Heilungsverlauf und löst vielfältige Komplikationen aus.

Um das therapeutische Ziel - die pathologisch wirksame Konzentration an bakteriellen Toxinen im Wundgebiet zu minimieren - gab es in der Strategie des modernen Wundmanagements bislang kaum wirksame therapeutische Ansatzpunkte. Auch eine Renaissance alter Verfahren wie Silberionen, Behandlung mit Maden, Behandlung mit Bienenhonig, Auflagen mit Aktivkohle usw. einzeln oder in Kombination auch mit modernen Antibiotika ist ohne nachweisbaren Nutzen hinsichtlich der Reduktion der pathologisch wirksamen Konzentration an bakteriellen Toxinen im Wundbereich geblieben.

Die der Erfindung zugrundeliegende Aufgabe war es daher, die Konzentration von Bakterientoxinen lokal durch geeignete Materialien nachhaltig zu reduzieren, ohne gleichzeitig das physiologische Milieu der Wunde, d. h. die natürlichen Konzentrationsverhältnisse der biologischen Mediatoren im Wundexsudat negativ zu beeinflussen oder deren Wirkung durch übermäßigen Flüssigkeitsentzug zu stören und damit der Wundheilung entgegen zu wirken. Es war das Ziel des Vorhabens, die Bakterientoxine von Beginn der Infektion an zu binden und dadurch zu entschärfen ohne das physiologische Wundmilieu nachhaltig zu ändern. Es war ferner das Ziel, die im Rahmen der Antibiose immer zu erwartenden unerwünschten Wirkungen der Bakterientoxine zu minimieren. Obgleich die Verwendung der beanspruchten Materialien hauptsächlich auf die örtliche Behandlung von Hautwunden zielt, gilt dies gleichermaßen für deren Anwendung in jeglichen infizierten Wundgebieten bis hin zu Abzessen in Körperhöhlen, Organen oder im Knochengewebe (Peritonitis, Pleuraempyem u. a.). Es ist zu erwarten, dass die Adsorption und Elimination von Bakterientoxinen u. U. auch anderer toxischer Substanzen vor Ort ähnlich dem systemisch angewandten SAFE-Konzept (EP 1 602 387), das "Outcome" betroffener Patienten hinsichtlich Morbidität und Mortalität deutlich verbessern wird.

Gelöst wird diese Aufgabe durch die Bereitstellung eines Materials zur Behandlung von lokalen bakteriellen Infektionen, insbesondere zur Prävention oder Behandlung von schädlichen Auswirkungen von lokalen bakteriellen Infektionen, welches dadurch gekennzeichnet ist, das es
a) ein Mittel zur Absorption von bakteriellen Lipopolysachariden (LPS) oder/und Lipoteichonsäuren (LTA) aufweist, welches gegebenenfalls in Kombination eingesetzt wird mit
b) einem Wundauflagematerial oder einem Trägermaterial für a), welches für die Anwendung im menschlichen Körper geeignet ist.

Das erfindungsgemäße Material ist konzipiert als Mehr-Komponenten-System: Einem Träger, der sich durch eine hohe Oberfläche auszeichnet, entweder verknüpft, vernetzt oder als Partikel und der durch eine spezifische Oberflächenbehandlung selektive, absorbierende Eigenschaften hinsichtlich bakterieller Toxine wie LPS und LTA besitzt. Ein solches Produkt könnte, als zusätzliche Komponente eingeführter auch neuerer Wundauflagen⁽¹³⁾ oder auch isoliert als Sofortbehandlung nach einer Operation zusätzlich in die Infekthöhle eingebracht werden.

Im Rahmen der vorliegenden Erfindung wurde festgestellt, dass bei der Behandlung von infizierten Wunden chromatographisch wirksame Adsorbentien, welche bakterielle Toxine binden, zum einen negative Einflüsse dieser Toxine, wie sie oben dargestellt sind, verhindern oder verringern können. Durch die adsorptive Bindung und damit Entfernung insbesondere der Endotoxine von gram-negativen Bakterien, der Lipopolysaccharide LPS, und auch der Toxine von gram-positiven Bakterien, der Lipoteichonsäure LTA, aus dem Wundmilieu wird es ermöglicht, im Bereich der Wunde eine erhebliche Verbesserung der pathophysiologischen Umstände herbeizuführen, sodass die natürliche Wundheilung weitestgehend ungehindert fortschreiten kann.

Ferner wurde festgestellt, dass mithilfe der erfindungsgemäßen Materialien zwar eine Entfernung der schädlichen bakteriellen Toxine in äußerst effektiver Weise ermöglicht wird, dagegen aber das physiologische Milieu der Wunde insbesondere im Hinblicke auf die Konzentrationsverhältnisse der biologischen Mediatoren im Wundexsudat, jedoch auch im Hinblick auf das chemische Milieu des Wundsekrets nicht oder äußerst geringfügig negativ beeinflusst wird.

Weiter konnte im Rahmen der vorliegenden Erfindung festgestellt werden, dass die Wundheilung auch dann positiv beeinflusst wird, wenn keine antimikrobiellen Substanzen eingesetzt werden. Ganz in Gegenteil zu der bisher überwiegend angewandten antibiotischen Behandlung von infizierten Wunden kann nämlich durch Maßnahmen der vorliegenden Erfindung vermieden werden, dass der Heilungsprozess durch die Freisetzung von bakteriellen Toxinen in der Folge einer Antibiose negativ beeinflusst wird. Soweit es dennoch angezeigt erscheint, Antibiotika lokal einzusetzen, kann bei Verwendung der erfindungsgemäßen Materialien vermieden werden, dass eine massive, lokale Freisetzung von bakteriellen Toxinen zum Übertritt dieser Toxine in den Blutstrom und damit in den gesamten Körper des Patienten führt, was die bekannte Folgekaskade auslöst, die über Aktivierung von Mediatoren teilweise autoimmun-destruktiv voranschreitet und zu septischen Erscheinungsformen bis hin zum septischen Schock und Tod des Patienten führen kann.

Im Rahmen der vorliegenden Erfindung kann jegliches Wundauflagematerial/Trägermaterial verwendet werden, welches für die betroffene Wundsituation geeignet ist. Für eine Anwendung an oberflächlichen Wunden kommen dazu alle gängigen Wundauflagematerialien in Betracht, welche die Heilung der Wunde fördern. Derartige Materialien sind keimfrei, bevorzugt steril und nicht flusend. Die Wundauflagematerialien sollten einen mechanischen Schutz der Wunde gewährleisten, wobei eine möglichst geringe Einschränkung der Mobilität des Patienten gewährleistet bleiben sollte. Außerdem sollten die verwendeten Wundmaterialien möglichst nicht am Wundgrund anhaften. Insbesondere sollten die verwendeten Wundauflagematerialien einen Gasaustausch ermöglichen und eine angemessene Exsudataufnahme gewährleisten, um eine unerwünschte Bildung von feuchten Kammern zu verhindern.

Prinzipiell sind sowohl Materialien zur feuchten, als auch zur trockenen Wundbehandlung einsetzbar.

Materialien zur trockenen Wundbehandlung sind dem Fachmann bekannt. Diese sogenannten "passiven Wundauflagen" decken die Wunde in erster Linie ab und saugen Exsudat auf, greifen jedoch nicht in den Wundheilungsprozess ein. Bei Verwendung derartiger Materialien zur trockenen Wundbehandlung wird das Wundsekret in das Wundauflagematerial aufgesaugt. Im Wundsekret enthaltene bakterielle Toxine können zur Adsorption (LPS und LTA) an Materialien der Erfindung gebunden werden.

Bevorzugt werden in Rahmen der vorliegenden Erfindung Materialien, die zu einer feuchten Wundbehandlung dienen. Diese Form der Verbandsmaterialien hat sich in der jüngeren Zeit besonders bewährt. Solche "aktiven" Wundauflagen fördern den Wundheilungsprozess, indem sie ein feuchtes Wundmilieu, das der Wundheilung besonders förderlich ist, aufrechterhalten.

Geeignete Wundauflagen sind dem Fachmann ebenfalls bekannt und umfassen unter anderem Schaumstoffe, Hydrogele, Hydrofasern, Hydrokolloide und Alginate.

Das feuchte Wundmilieu, welches bei Verwendung solcher Materialien erhalten bleibt, ermöglicht einen guten Kontakt zwischen dem Wundsekret und dem Mittel zur Adsorption der bakteriellen Toxine (LPS und LTA) und garantiert so die höchste Effektivität der Behandlung.

Neben der Behandlung von oberflächlichen Wunden sind auch interne Wunden wie postoperative Wund- und auch Abszesshöhlen für eine Behandlung mit dem erfindungsgemäßen Material geeignet. Bei derartigen Wundverhältnissen kann das erfindungsgemäße Material in die Wunde oder Höhle eingebracht und nach einer angezeigten Behandlungsdauer entfernt werden. Wesentlich ist, wie auch bei der Behandlung der oberflächlichen Wunden, dass die bakteriellen Toxine gebunden werden, sich nicht im Wundmilieu verteilen und nicht in die Blutbahn gelangen. Es ist hierbei auch denkbar, ein Trägermaterial in Form eines Drainagematerials auszugestalten, welches ohne erneuten operativen Eingriff später leicht durch Herausziehen entfernt werden kann.

Das erfindungsgemäß als Komponente a) eingesetzte Mittel zur Adsorption von bakteriellen Toxinen, LPS und LTA, besteht aus einem Adsorptionsträgermaterial, das durch eine chemische Modifikation eine hohe, spezifische Bindefähigkeit für LPS und LTA erhält.

Ähnliche Materialien sind zur Verwendung im Rahmen einer Blut- oder Plasmapherese bereits beschrieben. Verwiesen wird hiermit auf die entsprechende Offenbarung der Materialien in DE 44 35 612 und EP 1602387 B1.

Insbesondere ist es vorteilhaft, als Adsorptionsträgermaterial ein solches einzusetzen, welches eine größtmögliche Oberfläche für die chromatographische, adsorptive Bindung von bakteriellen Toxinen Adsorptionsreaktion bereitstellen kann. In einer bevorzugten Ausführungsform ist ein solches Trägermaterial ein Hohlfasermaterial. Erfindungsgemäße Hohlfasermaterialien gewährleisten eine selektive und effiziente Elimination von bakteriellen Toxinen und sie sind in aller Regel ohne Verlust oder Veränderung ihrer Eigenschaften mit Hitze oder gammaStrahlen sterilisierbar. Sie weisen außerdem die medizinisch erforderliche Bio- bzw. Hämokompatibilität auf und beeinträchtigen weder physiologische Regelkreise noch Schutzmechanismen, wie beispielsweise das Immun-, Komplement- oder Gerinnungssystem.

Der Fachmann kann gemäß diesem Anforderungsprofil geeignete HohlfaserAdsorptionsmaterialien auffinden. Im Prinzip können Hohlfasermaterialien verwendet werden, welche aus Polyamid, Polysulfon, Polyether, Polyethylen, Polypropylen, Polyester oder Derivaten oder/und Mischungen solcher Polymere hergestellt wurden. In besonders bevorzugter Ausführungsform der Erfindung bestehen die Hohlfasern aus Nylon.

Erfindungsgemäß geeignete Hohlfasermaterialien basieren z.B. auf Affinitätsmembranen, wie sie u.a. im U.S. Patent Nr. 5,053,133, 5,766,908, in der WO 96/22316 beschrieben sind. Diese Membrangrundmaterialien können nach an sich bekannten Methoden und insbesondere durch Pfropfpolymerisation modifiziert werden, beispielhaft sei in diesem Zusammenhang auf die WO 96/22316 hingewiesen, in der ein entsprechendes Verfahren beschrieben ist. Andere Derivatisierungsverfahren für entsprechende Polymermaterialien, welche zur Herstellung von Hohlfaseradsorptionsmaterialien geeignet sind, sind dem Fachmann ebenfalls bekannt und im Rahmen der vorliegenden Erfindung anwendbar.

Außer in Form von Hohlfasermaterialien können die genannten geeigneten Adsorptionsträgermaterialien auch in anderer Ausgestaltung eingesetzt werden, wie z.B. als Flachmembranen, poröse Partikel, Schwämme oder Filtermaterialien. Wesentlich für die Wirksamkeit der erfindungsgemäßen Materialien ist, dass ihre chemische Modifikation eine gute adsorptive Bindung von Bakterientoxinen erbringt. Ferner ist es von Vorteil, wenn eine gute Benetzung der Materialien durch das Wundsekret gewährleistet ist und die Materialien im Wundsekret eine möglichst große Oberfläche bieten. Die (Filter-) Materialien müssen also grundsätzlich für chromatographische Anwendungen geeignet sein. Am besten geeignet habe sich zu diesem Zwecke Hohlfasern, Flachmembranen und poröse Partikel erwiesen.

Moderne und geeignete Membranen sind z.B. in einer Übersicht von Liu et al. (Journal of Biomedical Materials Research A/Aug. 2010, Vol. 94A, Issue 2, S. 499) beschrieben.

Weitere besonders gut geeignete chromatographische Trägermaterialien sind im Rahmen der vorliegenden Erfindung solche, wie sie in der EP 1 348 999 beschreiben sind. Diese Materialien weisen Ausschlussgrößen von kleiner 15.000 Da auf. LPS und LTA sind sehr große Biopolymere, die bis zu einige Millionen Dalton Molekulargewicht aufweisen können. Die toxische Substanz im Rahmen von LPS und LTA ist aber jeweils ein Lipid mit einem Molekulargewicht von etwa 1.500 Da. Bei LPS handelt es sich um Lipid A, welches an die Kopfseite der veresterten Zuckerpolymere von LPS gebunden ist. Bei LTA ist die eigentliche toxische Substanz ebenfalls ein Lipid, das gleiche chemische, ionische und hydrophobe Eigenschaften wie Lipid A aufweist. Lipoteichonsäuren bestehen aus einer hydrophilen Kette aus Alditolphosphaten und einem Glycolipid, welches die Kette in der Membran der Bakterien verankert.

Bei der Wundinfektion kommt es in der Regel zu einem enzymatischen Abbau von LPS und LTA, bei dem die toxischen Lipide freigesetzt werden und so ihre negativen Auswirkungen verursachen. Es sind vornehmlich Hydrolyasen, die für diesen Abbau verantwortlich sind.

Um zu vermeiden, dass durch das Adsorptionsmaterial für die Wundheilung wichtige Makromoleküle aus dem Wundsekret entfernt werden, ist es von großem Vorteil, wenn in das eingesetzte Material keine Makromoleküle eindringen können, es aber dennoch in der Lage ist, das toxische Prinzip von LPS und LTA - die jeweiligen Lipide - chromatographisch aufzunehmen und über die immobilisierten Liganden Modifikation des Materials selektiv zu binden.

Dies wird ermöglicht bei Verwendung von Materialien mit Ausschlussgrößen von kleiner als 100.000 Da, vorzugsweise kleiner 50.000 Da. und besonders bevorzugt kleiner 15.000 Da.

Das erfindungsgemäße Mittel zur Absorption von LPS und LTA weist als chemische Modifikation eine solche auf, die es ermöglicht, LPS und LTA selektiv und effektiv zu binden und somit aus dem Wundsekret aufzunehmen bzw. aus dem reaktiven Wundraum zu entfernen. Das Trägermaterial ist hierzu modifiziert, weist also Liganden auf, welche LPS und LTA binden. Bevorzugt wird mittels einer Propfpolymerisation eine Gruppierung eingebracht, die gute Bindungseigenschaften für LPS oder/und LTA aufweist.

Derartige Materialien sind z.B. aus EP 1 602 387 bekannt.

Als wiederum besonders bevorzugt hat es sich erwiesen, zur Bindung von LPS oder LTA bzw. deren Lipide Anionenaustauschergruppierungen aufzupfropfen. Derartige Anionenaustauschergruppierungen sind besonders vorteilhaft als längere Ketten ausgestaltet mit einer Vielzahl von kationischen Gruppen, als sogenannte Tentakel. Derartige tentakelartige Fortsätze auf dem Grundmaterial sind fähig, mehrere LPS- bzw. LTA-Moleküle zu binden, wodurch die Effizienz des Hohlfasermaterials nochmals gesteigert werden kann. Für diese Modifikation des Hohlfasermaterials mittels Tentakel werden vorzugsweise synthetische oder/und halbsynthetische oder/und natürliche Polykationenketten eingesetzt, wobei diese Ketten in linearer oder verzweigter Form vorliegen können. Derartige Kationen- bzw. Polykationenketten weisen bevorzugt tertiäre oder/und quartäre Amingruppen auf.

Bevorzugte Anionenaustauschergruppen auf den Hohlfasermaterialien schließen Di- oder Trialkylaminoalkyl-, Di- oder Trialkylaminoaryl-, Di- oder Triarylaminoalkyl-, Di- oder Triarylaminoaryl-, Di- oder Trialkylammoniumalkyl-, Di- oder Triarylammoniumalkyl-, Di- oder Triarylammoniumaryl- und Di- oder Trialkylammoniumaryl-Reste ein. Des Weiteren sind Polymere aus positiv geladenen oder tertiäre oder quartäre Aminogruppen enthaltenden Aminosäuren, wie Polylysin, Polyarginin oder Polyhistidin oder Mischpolymere oder Derivate hiervon im Rahmen der Erfindung als Anionenaustauschermaterialien geeignet, ebenso Polyethylenimin.

Eine bevorzugte Anionenaustauschergruppierung enthält Trimethylbenzylammonium-Reste. Eine entsprechende beispielhafte Verbindung, welche als Anionenaustauschergruppierung verwendet werden kann, ist Cholestyramin (Poly(trimethylammoniomethylstyrolchlorid-co-divinylbenzol)).

In ganz besonders bevorzugten Ausführungsformen der Erfindung enthält das erfindungsgemäße Mittel ein mit Diethylaminoalkyl- bzw. Diethylaminoaryl-Resten modifiziertes Polyamid-Hohlfasermaterial, insbesondere Diethylaminoethyl-Polyamid.

In Abhängigkeit von dem verwendeten Wundauflagematerial oder Trägermaterial als Komponente b) kann das erfindungsgemäße Material als einschichtiges Material oder als mehrschichtiges Material ausgestaltet sein.

Bei einer Ausgestaltung als einschichtiges Material werden das Wundauflagematerial bzw. das Trägermaterial und das Mittel zur Adsorption von LPS und LTA als eine gemischte Schicht eingesetzt. Dabei ist es möglich, das Mittel zur Adsorption von LPS und LTA in irgendeiner geeignet erscheinenden Form im Wundauflagematerial/Trägermaterial darzustellen. Je nach den Bedürfnissen der einzelnen zu versorgenden Wunde kann anstelle von verschiedenen Materialien für Wundauflage/Schutzmaterial und Adsorptionsmaterial auch eine Schicht eingesetzt werden, in welcher das Adsorptions-Trägermaterial, also z.B. das Hohlfasermaterial oder die Flachmembran, selbst das Wundauflagematerial darstellt. Eine derartige Ausgestaltung ist auch insbesondere bei Anwendung in Körperhohlräumen des Patienten einsetzbar. Eine Abdeckung durch ein den Wundbereich schützendes Material kann dann, falls gewünscht, insbesondere bei oberflächlichen Wunden, mithilfe einer darüber angeordneten Schicht eines beliebigen anderen geeigneten Wundverbandsmaterials erfolgen.

Ebenso ist es möglich, eine Schicht aus einem bekannten Wundmaterial in Kombination mit dem Mittel zur Adsorption zu kombinieren, indem eine gemischte Schicht aus beiden Materialien erzeugt wird.

In einer weiteren bevorzugten Ausführungsform wird ein mehrschichtiges Material eingesetzt. Hierbei ist es zum einen möglich, eine oder mehrere Schichten bekannter Wundverbandsmaterialien mit einer oder mehreren Schichten des Adsorptionsmittels zu kombinieren. In dieser Ausgestaltung ist es lediglich wichtig, dass die Materialien ausreichend Saugfähigkeit und Durchlässigkeit für Wundsekret aufweisen, sodass die Kapazitäten des Adsorptionsmaterials zur Bindung von LPS und LTA genutzt werden können. Insbesondere ist zu vermeiden, dass durch ein Material, welches nicht die äußerste Schicht darstellt, eine Barrierewirkung für das Wundsekret verursacht wird.

Die erfindungsgemäßen Materialien ermöglichen eine effektive Entfernung von bakteriellen Toxinen aus Wundsekret. Es gelingt damit, diese für die Wundheilung, aber auch im Hinblick auf septische Reaktionen schädlichen Substanzen einfach und effektiv zu beherrschen. Nach Verbandswechsel sind die gebundenen bakteriellen Toxine aus dem Wundmilieu entfernt und ein neuer Verband kann bei fortbestehender bakterieller Besiedelung wiederum freigesetzte Toxine adsorbieren und unschädlich machen.

Die erfindungsgemäßen Materialien können aufgrund ihrer guten Bindungseigenschaften für LPS und LTA auch in Kombination mit Antibiotika oder anderen bakteriziden Wirkstoffen eingesetzt werden, ohne dass die Abtötung von Bakterien durch diese Wirkstoffe und die damit einhergehende, oben beschriebene Freisetzung der Toxine Schäden verursacht. Somit kann eine Therapie mit Antibiotika oder anderen bakteriziden Mitteln durch die erfindungsgemäßen Materialien unterstützt und damit die Wundheilung deutlich verbessert werden.

Ein weiterer Gegenstand der vorliegenden Anmeldung ist die Verwendung der erfindungsgemäßen Materialien zur Behandlung von lokalen bakteriellen Infektionen, insbesondere von oberflächlichen oder inneren Wunden.

In einer Ausführungsform der Erfindung wird dabei das erfindungsgemäße Material ohne weitere bakterizide Wirkstoffe eingesetzt.

In einer anderen Ausführungsform der Erfindung wird das erfindungsgemäße Material gemeinsam mit bakteriziden Mitteln eingesetzt. Die bakteriziden Mittel können dabei in das erfindungsgemäße Material integriert sein, indem z.B. das Material mit einer entsprechenden Lösung getränkt wurde.

Ebenfalls möglich ist es, zuerst die Wunde mit bakteriziden Wirkstoffen in geeigneter Anwendungsform zu behandeln und dann die behandelte Wunde mit dem erfindungsgemäßen Material abzudecken.

Die Erfindung soll durch das folgende Beispiel veranschaulicht werden.

### Beispiel

### Adsorption und Elimination von LPS und LTA aus simuliertem Wundexsudat in Gegenwart von Zytokinen und eines Wachstumsfaktors

Das simulierte Wundexsudat bestand aus Humanalbumin (2 Gewichtsprozent), Calciumchlorid (0.02 M), Natriumchlorid (0.4 M) und Trismethylamin (0.08 M), gelöst in destilliertem Wasser. Der pH Wert wurde auf pH 7.5 eingestellt.

Zu dem simulierten Wundexsudat wurden folgende bakteriellen Toxine, humane Zytokine und ein repräsentativer Wachstumsfaktor hinzugefügt:
A. Lipopolysaccharide (LPS, 2 EU/mL, E.coli 055: B5 Endotoxin; Fa. BioWhitaker)
B. Lipoteichonsäuren (LTA, 1 µg/mL, Streptococcus pyogenes; Fa. Sigma Aldrich)
C. Recombinanter humaner Tumor-Nekrose-Faktor alpha (rh TNF-α, 10 pg/ml; Fa. Biomol)
D. Recombinantes humanes Interleukin-6 (rh IL-6, 50 pg/mL; Fa. Biomol)
E. Recombinanter humaner Platelet-Derived-Growth-Factor A (rhPDGF A; 50 ng/ml; Fa. Biomol

Ein Aliquot (5mL) des simulierten bzw. mit bakteriellen Toxinen und humanen Zytokinen sowie mit einem Wachstumsfaktor versetzten Wundexsudats wurde mit jeweils 100 mg der nachstehend aufgeführten Adsorbentien versetzt, für 4 Stunden bei 37°C inkubiert und danach bei 1000g abzentrifugiert.

### Verwendete Adsorbentien:

1. Cholestyramin (Quantalan, Bristol Meyer Squibb)
2. LiChrospher XDS DEAE (entsprechend EP 1019188 A1; E.Merck KGaA)
3. Toyopearl DEAE (M-grade; Tosoh Bioscience)
4. DEAE-modifizierte Polyamid-Hohlfaser (entsprechend DE 10258944 A1; B.Braun Melsungen AG)

Die verwendeten Adsorbentien (1-4) besitzen die nachfolgend aufgeführten, mittleren Porendurchmesser. 1) 2 nm; 2) 6 nm; 3) 100nm; 4) 500µm.

Die dem simulierten Wundexsudat hinzugefügten Stoffe (A-E) haben folgende Molekulargewichte: A und B) gemeinsame Lipid A Untereinheit ∼ 2000 Dalton; C) ∼ 50000 Dalton; D) ∼ 25000 Dalton; E) ∼ 30000 Dalton.

### Bestimmungsmethoden:

LPS wurde mit Hilfe des chromogenen kinetischen Limulus-Amoebocyten-Lysat Tests (LAL-QCL, Fa. Cambrex) bestimmt.
Der Nachweis von LTA wurde indirekt mit Hilfe eines Vollblut-Stimulationstests durchgeführt. Hierzu wurde frisch gewonnenes heparinisiertes Vollblut eines gesunden Spenders mit dem unbehandelten bzw. behandelten präpariertem Wundexsudat im Verhältnis 6/1 (v/v) versetzt und für 24 Stunden bei Raumtemperatur inkubiert. Danach wurde nach Zentrifugation (3000 g für 10 min.) und Gewinnung der entsprechenden Plasmafraktion, die durch die LTA stimulierte Interleukin-6 (IL-6) Biosyntheserate bestimmt. Rekombinantes humanes Interleukin-6 (IL-6) und rh PDGF-A wurde mit Hilfe eines entsprechenden ELISA-Tests (Fa.Biomol GmbH) quantifiziert.
Die Konzentration des rekombinanten humanen TNF-α wurde über einen EIA-Kit (Fa. Biomol GmbH) bestimmt.

### Ergebnisse:

Nach Inkubation mit dem präparierten Wundexsudat und Entfernung der verwendeten Adsorbentien durch Zentrifugation, wurde in dem jeweiligen Überstand die prozentuale Abreicherung (Eliminationsrate) der zugefügten Komponenten (A-E, d.h. bakterielle Toxine, Zytokine,und Wachstumsfaktor) bestimmt.

Es ergaben sich folgende Eliminationsraten [%]:

| | **Adsorber** | | | |
|---|---|---|---|---|
| | **1** | **2** | **3** | **4** |
| LPS | 95 | 90 | 90 | 85 |
| LTA | 95 | 85 | 90 | 90 |
| TNF | 10 | 10 | 75 | 70 |
| IL-6 | 10 | 15 | 90 | 80 |
| PDGF-A | 5 | 5 | 85 | 80 |

### Literatur:

1. J. Heinlin et al. Wundheilung - Therapeutische Interventionen Hautarzt 2010, 61:611-628
2. Y. Winkler Endotoxin-Bindung, bakteriozide Wirksamkeit und Zytotoxizität silberhaltiger Wundauflagen Inauguraldissertation, Ernst-Moritz-Arndt-Universität Greifswald, 2010
3. M. Schäffer, H.-D. Becker Immunregulation der Wundheilung Chirurg 1999, 70:897-908
4. H. Lippert Wundatlas. Kompendium der komplexen Wundbehandlung Georg Thieme Verlag KG, Stuttgart / New York, 2006
5. M. Voshege et al. Was ist evidenzbasiert in der Behandlung chronischer Wunden? Gefäßchir 2003, 8:269-276,
6. P. K. Baier et al. Subcutaneous Redon drains do not reduce the incidence of surgical site infections after laparotomy. A randomized controlled trial on 200 patients Int J Colorectal Dis 2010, 25:639-643
7. S. Trambe et al. In vitro evaluation of the risk of developing bacterial resistance to antiseptics Atimicrob Chemother 2001, 47:589-598
8. P. Appelgren et al. A prospective study of infections in burn patients Burns 2002, 28:39-46
9. G. Müller et al. Biocompatibility index of antiseptic agents by parallel assessment of antimicrobial activity and cellular cytotoxicity J Antimicrob Chemother 2008, 61:1281-1287
10. W. Lineaweaver et al. Topical antimicrobial toxicity Arch Surg 1985, 120:267-270
11. R. Stone Search for sepsis drugs goes on despite past failures Science 1994, 264:365-367
12. Z. Metzger et al. The effect of bacterial endotoxin on the early tensile strength of healing surgical wounds J Endod 2002, 28:30-33
13.Xin Liu et al. In vivo wound healing and antibacterial performances of electrospun nanofibre membranes J Biomed Mater Res A. 2010, 94:499-508

## Patentansprüche

1. Mittel zur Verwendung bei der Behandlung von lokalen bakteriellen Infektionen, insbesondere durch Prävention oder Behandlung von schädlichen Auswirkungen von lokalen bakteriellen Infektionen, durch Bindung bakterieller Toxine aus infizierten Wunden, welches **dadurch gekennzeichnet ist, dass** es
a) ein Mittel zur Adsorption von bakteriellen Lipopolysacchariden (LPS) oder/und Lipoteichonsäuren (LTA) aufweist, welches Anionenaustauschergruppierungen aus Polykationen und hydrophobe Eigenschaften aufweist, wobei die Anionenaustauschergruppierungen ausgestaltet sind als Polykationenketten aus synthetischen oder/und halbsynthetischen oder/und natürlichen Polykationenketten, welche in linearer oder verzweigter Form vorliegen, und gegebenenfalls in Kombination eingesetzt wird mit
b) einem Wundauflagematerial oder einem Trägermaterial für a), welches für die Anwendung im menschlichen Körper geeignet ist,

2. Mittel nach Anspruch 1,
**dadurch gekennzeichnet, dass** das Wundauflagematerial zur Anwendung an oberflächlichen Wunden ausgestaltet ist.

3. Mittel nach Anspruch 1,
**dadurch gekennzeichnet, dass** das Wundauflagematerial zur Anwendung bei internen Wunden, insbesondere chirurgischen Wunden sowie abgekapselten Infektionen und Abszessen ausgestaltet ist.

4. Mittel nach einem der vorhergehenden Ansprüche,
**dadurch gekennzeichnet, dass** das Wundauflagematerial Wundflüssigkeit und Wundsekret adsorbiert.

5. Mittel nach Anspruch 3 oder 4,
**dadurch gekennzeichnet, dass** das Wundauflagematerial als Drainagematerial ausgestaltet ist.

6. Mittel nach einem der vorhergehenden Ansprüche,
**dadurch gekennzeichnet, dass** das Mittel ein mehrschichtiges Material ist.

7. Mittel nach einem der vorhergehenden Ansprüche,
**dadurch gekennzeichnet, dass** das Mittel zur Adsorption von bakteriellen Lipopolysacchariden und Lipoteichonsäuren auf mindestens eine Lage des Wundauflagematerials aufgebracht ist.

8. Mittel nach einem der vorhergehenden Ansprüche,
**dadurch gekennzeichnet, dass** das Mittel zur Adsorption von bakteriellen Lipopolysacchariden und Lipoteichonsäuren als mindestens eine eigene Schicht innerhalb eines mehrschichtigen Wundauflagematerials ausgestaltet ist.

9. Mittel nach einem der vorhergehenden Ansprüche,
**dadurch gekennzeichnet, dass** das Mittel zur Adsorption von bakteriellen Lipopolysacchariden und Lipoteichonsäuren auf ein Wunddrainagematerial oder eine Wundtamponage als Trägermaterial aufgebracht ist.

10. Mittel nach einem der vorhergehenden Ansprüche,
**dadurch gekennzeichnet, dass** die Anionenaustauschergruppierungen Di- oder Trialkylaminoalkyl-, Di- oder Trialkylaminoaryl-, Di- oder Triarylaminoalkyl-, Di- oder Triarylaminoaryl-, Di- oder Trialkylammoniumalkyl-, Di- oder Triarylammoniumalkyl-, Di- oder Triarylammoniumaryl-, oder Di- oder Trialkylammoniumaryl-Reste enthalten oder Polymere aus positiv geladenen oder tertiäre oder quartäre Aminogruppen enthaltenden Aminosäuren, wie Polylysin, Polyarginin oder Polyhistidin oder Mischpolymere oder Derivate hiervon oder Polyethylenimin sind.

11. Mittel nach einem der vorhergehenden Ansprüche,
**dadurch gekennzeichnet, dass** die Anionenaustauschergruppierung Trimethylbenzylammonium-Reste enthält.

## Claims

1. Product for use in the treatment of local bacterial infections, in particular by preventing or treating harmful effects of local bacterial infections, by binding bacterial toxins from infected wounds, which product is **characterised in that**:
a) it comprises a product for adsorbing bacterial lipopolysaccharides (LPS) and/or lipoteichoic acids (LTA), which product comprises anion-exchanger groupings of polycations and has hydrophobic properties, the anion-exchanger groupings being in the form of polycation chains of synthetic and/or semi-synthetic and/or natural polycation chains which are present in linear or branched form and are optionally used in combination with
b) a wound-dressing material or a carrier material for a), which material is suitable for use in the human body.

2. Product according to claim 1, **characterised in that** the wound-dressing material is designed to be used on surface wounds.

3. Product according to claim 1, **characterised in that** the wound-dressing material is designed to be used for internal wounds, in particular surgical wounds and encapsulated infections and abscesses.

4. Product according to any of the preceding claims, **characterised in that** the wound-dressing material adsorbs wound fluid and wound secretions.

5. Product according to either claim 3 or claim 4, **characterised in that** the wound-dressing material is in the form of drainage material.

6. Product according to any of the preceding claims, **characterised in that** the product is a multi-layer material.

7. Product according to any of the preceding claims, **characterised in that** the product for adsorbing bacterial lipopolysaccharides and lipoteichoic acids is applied to at least one layer of the wound-dressing material.

8. Product according to any of the preceding claims, **characterised in that** the product for adsorbing bacterial lipopolysaccharides and lipoteichoic acids is in the form of at least one individual layer within a multi-layer wound-dressing material.

9. Product according to any of the preceding claims, **characterised in that** the product for adsorbing bacterial lipopolysaccharides and lipoteichoic acids is applied to a wound-drainage material or a wound tamponade as a carrier material.

10. Product according to any of the preceding claims, **characterised in that** the anion-exchanger groupings contain di- or trialkylamino alkyl groups, di- or trialkylamino aryl groups, di- or triarylamino alkyl groups, di- or triarylamino aryl groups, di- or trialkylammonium alkyl groups, di- or triarylammonium alkyl groups, di- or triarylammonium aryl groups, or di- or trialkylammonium aryl groups, or polymers from amino acids containing positively charged or tertiary or quaternary amino groups, such as polylysine, polyarginine or polyhistidine or copolymers of derivatives thereof, or polyethylenimine.

11. Product according to any of the preceding claims, **characterised in that** the anion-exchanger grouping contains trimethylbenzylammonium groups.

## Revendications

1. Moyen destiné à être utilisé dans le traitement d'infections bactériennes locales, en particulier par la prévention ou le traitement de conséquences nuisibles d'infections bactériennes locales, par la liaison de toxines bactériennes en provenance de plaies infectées, qui est **caractérisé en ce**
a) **qu'**il présente un moyen destiné à l'adsorption de lipopolysaccharides (LPS) et/ou d'acides lipotéichoïques bactériens (LTA), qui présente des groupements échangeurs d'anions de polycations et des propriétés hydrophobes, dans lequel les groupements échangeurs d'anions se présentant sous la forme de chaînes polycationiques, de chaînes polycationiques synthétiques et/ou semi-synthétiques et/ou naturelles qui se présentent sous forme linéaire ou ramifiée, et qui est éventuellement mis en oeuvre en combinaison avec
b) un matériau de pansement ou un matériau support pour a), qui est approprié pour une utilisation dans le corps humain.

2. Moyen selon la revendication 1,
**caractérisé en ce que** le matériau de pansement se présente sous une forme convenant pour une utilisation sur des plaies superficielles.

3. Moyen selon la revendication 1,
**caractérisé en ce que** le matériau de pansement se présente sous une forme convenant pour une utilisation avec des plaies internes, en particulier des plaies chirurgicales, ainsi que des infections encapsulées et des abcès.

4. Moyen selon l'une des revendications précédentes,
**caractérisé en ce que** le matériau de pansement adsorbe le liquide de la plaie et les sécrétions de la plaie.

5. Moyen selon la revendication 3 ou 4,
**caractérisé en ce que** le matériau de pansement se présente sous la forme d'un matériau de drainage.

6. Moyen selon l'une des revendications précédentes,
**caractérisé en ce que** le moyen est un matériau multicouche.

7. Moyen selon l'une des revendications précédentes,
**caractérisé en ce que** le moyen pour l'adsorption des lipopolysaccharides et des acides lipotéichoïques bactériens est appliqué sur au moins une couche du matériau de pansement.

8. Moyen selon l'une des revendications précédentes,
**caractérisé en ce que** le moyen pour l'adsorption des lipopolysaccharides et des acides lipotéichoïques bactériens se présente sous la forme d'au moins une couche distincte à l'intérieur d'un matériau de pansement multicouche.

9. Moyen selon l'une des revendications précédentes,
**caractérisé en ce que** le moyen pour l'adsorption des lipopolysaccharides et des acides lipotéichoïques bactériens est appliqué sur un matériau de drainage de la plaie ou sur un matériau pour tamponner la plaie en tant que matériau support.

10. Moyen selon l'une des revendications précédentes,
**caractérisé en ce que** les groupements échangeurs d'anions contiennent des restes di- ou trialkylaminoalkyle, di- ou trialkylaminoaryle, di ou triarylaminoalkyle, di- ou triarylaminoaryle, di- ou trialkylammoniumalkyle, di- ou triarylammoniumalkyle, di- ou triarylammoniumaryle ou di- ou trialkylammoniumaryle ou sont des polymères d'acides aminés chargés positivement ou contenant des groupes amino tertiaires ou quaternaires tels que la polylysine, la polyarginine ou la polyhistidine ou des copolymères ou des dérivés de ceux-ci, ou de polyéthylèneimine.

11. Moyen selon l'une des revendications précédentes,
**caractérisé en ce que** le groupement échangeur d'anions contient des restes triméthylbenzylammonium.
